# EUROPEAN PATENT SPECIFICATION

(11) **EP 2 956 065 B1**
(45) Date of publication and mention of the grant of the patent: **17.06.2020**
(21) Application number: 14707551.9
(22) Date of filing: 12.02.2014
(51) Int. Cl.: A61B 6/03, A61B 6/00, A61B 8/08, A61B 8/12, A61B 8/14, A61B 8/00

(54) **APPARATUS FOR IMAGE FUSION BASED PLANNING OF C-ARM ANGULATION FOR STRUCTURAL HEART DISEASE**
VORRICHTUNG ZUR AUF EINER BILDFUSION BERUHENDEN PLANUNG EINER C-ARM-ABWINKELUNG FÜR STRUKTURELLE HERZKRANKHEIT
APPAREIL DESTINÉS À LA PLANIFICATION BASÉE SUR LA FUSION D'IMAGES D'UNE ANGULATION SUR ARCEAU POUR UNE MALADIE CARDIAQUE STRUCTURELLE

(30) Priority: 13.02.2013 US 201361764141 P; 11.02.2014 US 201414177340
(43) Date of publication of application: 23.12.2015
(73) Proprietor: Siemens Healthcare GmbH, 91052 Erlangen (DE)
(72) Inventor: JOHN, Matthias, 90429 Nürnberg (DE); SZUCS, Murrill Michael, Snoqualmie, Washington 98065 (US); IONASEC, Razvan, 90403 Nuernberg (DE); NOETTLING, Alois, 91278 Pottenstein (DE)
(74) Representative: Isarpatent
(86) International application number: PCT/US2014/015928
(87) International publication number: WO 2014/126955

(56) References cited:
- WO-A1-2011/086475
- DE-A1-102005 032 523
- DE-U1-202008 018 167
- US-A1- 2011 052 026
- US-A1- 2011 222 750
- US-A1- 2012 296 202
- US-A1- 2012 323 545

## Description

### BACKGROUND OF THE INVENTION

The present invention relates to medical imaging of the heart, and more particularly, to using medical images of the heart to determine an angulation of a C-arm image acquisition system for an intervention to treat structural heart disease.

Many devices are available or under development for minimally invasive treatment of structural heart disease suing catheters instead of more invasive surgery using a heart-lung machine. This provides the opportunity to treat sicker patients and also opens the field to interventional cardiologists. Aortic valve disease is the most common valvular disease in developed countries, and has the second highest incidence among congenital valvular defects. Implantation of an artificial valve (i.e., valve prosthesis) is often necessary to replace a damaged natural valve. Transcatheter Aortic Valve Implantation (TAVI) is a minimally invasive intervention to replace the aortic valve. In TAVI, an aortic valve prosthesis is inserted via a catheter and X-ray imaging is used to support a physician in positioning and deployment of the valve prosthesis. In particular, during a valve implantation surgery, 2D fluoroscopic images (X-ray images) are often captured in real time using a C-arm image acquisition system to provide guidance to the physician.

In order to achieve good intervention results, it is desirable to have a dedicated C-arm angulation that provides an optimal view of the area of interest. For example, for TAVI, an x-ray view that is perpendicular to the aortic root is desirable. In conventional valve implantation procedures, physicians typically select an angulation for a C-arm X-ray device by iteratively acquiring angiograms using a contrast agent. From each angiogram, a physician manually predicts a good angulation until an appropriate angulation for the valve implantation procedure is selected. This selection process typically requires at least 2-3 iterations. Accordingly, this selection process typically requires a large amount of contrast agent and is time consuming.

Document DE 10 2005 032 523 A1 discloses a method for pre-interventional planning of a 2D fluoroscopy projection for an interventional entry using a fixed instrument.

Document US 2012 / 0 323 545 A1 discloses a device for planning a transcatheter aortic valve implantation.

Document US 2011 / 0 222 750 A1 discloses a method for guiding transcatheter aortic valve implantations. Document US 2012/0296202 discloses a method and system for registering ultrasound images to Xray fluoroscopic images.

### BRIEF SUMMARY OF THE INVENTION

The present invention provides system for image fusion based planning of C-arm angulation for structural heart disease interventions with the features of the independent claims.

In one embodiment of the present invention, a 3D ultrasound image including a cardiac region is received. The 3D ultrasound image is registered to a 3D coordinate system of a C-arm image acquisition system. A cardiac structure of interest is detected in the registered 3D ultrasound image. An angulation of the C-arm image acquisition system is determined based on the detected structure of interest in the registered 3D ultrasound image.

These and other advantages of the invention will be apparent to those of ordinary skill in the art by reference to the following detailed description and the accompanying drawings.

### BRIEF DESCRIPTION OF THE DRAWINGS

FIG. 1 illustrates a method for determining an optimal angulation of a C-arm image acquisition device for a cardiac intervention;
FIG. 2 illustrates a method of registering a 3D ultrasound image to a coordinate system of a C-arm image acquisition device;
FIG. 3 illustrates probe detection in a 2D X-ray image;
FIG. 4 illustrates a method for estimating the pose;
FIG. 5 illustrates a method of refining an estimated pose of an ultrasound probe;
FIG. 6 illustrates a probe in 3D;
FIG. 7 illustrates a 3D ultrasound image mapped to the 3D local coordinate system of a C-arm image acquisition device;
FIG. 8 illustrates exemplary C-arm angulation planning based on the annulus plane of the mitral valve; and
FIG. 9 is a high level block diagram of a computer capable of implementing the present invention.

### DETAILED DESCRIPTION

The present invention is directed to a system for image fusion based C-arm angulation planning for interventions to treat structural heart disease. Embodiments are described herein to give a visual understanding of the method for determining an optimal angulation. A digital image is often composed of digital representations of one or more objects (or shapes). The digital representation of an object is often described herein in terms of identifying and manipulating the objects. Such manipulations are virtual manipulations accomplished in the memory or other circuitry / hardware of a computer system. Accordingly, it is to be understood that embodiments of the present invention may be performed within a computer system using data stored within the computer system.

Embodiments of the present invention utilize ultrasound and x-ray fusion to determine an optimal C-arm angulation for catheter based procedures to treat structural heart disease. Embodiments of the present invention utilize intra-operative images and therefore reflect the current state of the patient. Embodiments of the present do not require any extra operating room setting like in intra-operative 3D X-ray imaging. Embodiments of the present invention reduce the amount of radiation and contrast agent exposure required for a patient as compared to conventional techniques for determining a C-arm angulation.

In an advantageous embodiment, the image fusion based method is used to determine an optimum C-arm angulation for Transcatheter Aortic Valve Implantation (TAVI). The method described here can similarly be used to determine optimum C-arm angulations for other procedures as well, including but not limited to mitral valve repair by MitraClip, transcatheter mitral valve implantation, left atrial appendage closure, and paravalvular leak closure.

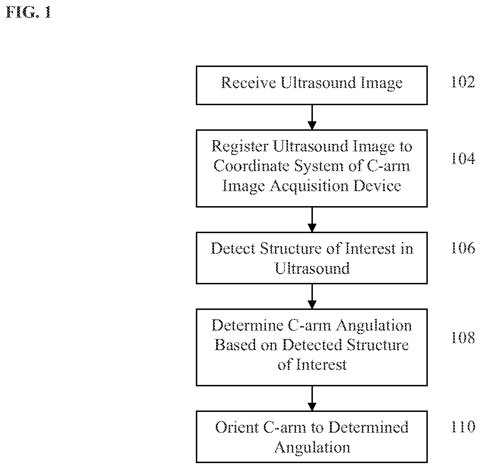

FIG. 1 illustrates a method for determining an optimal angulation of a C-arm image acquisition device for a cardiac intervention. The method of FIG. 1 transforms medical image data representing a cardiac region of a patient to detect and visualize various anatomic features of the patient and to determine the optimal angulation for C-arm X-ray acquisition for the cardiac intervention. In an exemplary embodiment, the method of FIG.1 can be used to determine a C-arm angulation for TAVI. The method of FIG. 1 can also be used to determine a C-arm angulation for other cardiac intervention procedures, such as mitral valve repair, transcatheter mitral valve implantation, atrial appendage closure, and paravalvular leak closure.

At step 102, an ultrasound image of the region of interest is received. According to an advantageous implementation, the ultrasound image is a 3D ultrasound image acquired using transesophageal echo (TEE) or intracardiac echo (ICE). The 3D ultrasound image can be an intraopertative image acquired using an ultrasound probe at the beginning of the cardiac procedure. The 3D ultrasound image can be received directly from the ultrasound probe in real time during the cardiac procedure. In the application to TAVI, the ultrasound image is a 3D ultrasound image of the aortic root acquired before inserting the valve prosthesis via a catheter.

At step 104, the ultrasound image is registered to a coordinate system of the C-arm image acquisition device. In particular, the ultrasound image is registered to the "table" coordinate system of the C-arm image acquisition device. The table coordinate system is a mechanical coordinate system that is oriented with respect to the table of the C-arm device and thus remains constant even as the C-arm portion of the C-arm image acquisition device is rotated with respect to the table.

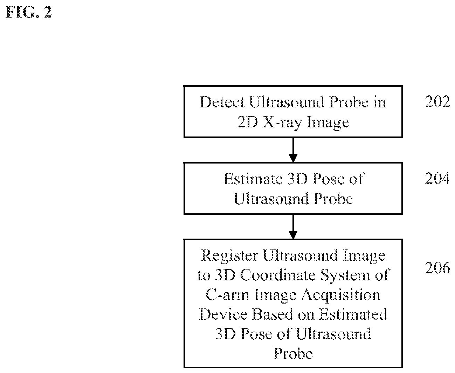

In an exemplary embodiment, a 2D x-ray image including the ultrasound probe (which is located within the patient's body) can be acquired using the C-arm image acquisition device at approximately the same time as the ultrasound image is acquired and the 3D position of the ultrasound probe relative to the 2D x-ray image can be determined, and from this 3D position the position of the ultrasound image relative to the table coordinate system of the C-arm image acquisition device can be derived. FIG. 2 illustrates a method of registering a 3D ultrasound image to a coordinate system of a C-arm image acquisition device. The method of FIG. 2 may be used to implement step 104 of FIG. 1. This method of FIG. 2 is described in greater detail in United States Published Patent Application No. 2013/02379780.

At step 202, the ultrasound probe is detected in the 2D X-ray image. According to an advantageous implementation, a learning based method can be used for probe detection. Learning based methods are robust to noise and capable of handling large variations in appearance. Unlike matching or similarity measures, learning based methods are trained on a set of manually annotated or synthetically generated training data. In particular, a probe detector is trained using a learning based method offline prior to the cardiac intervention procedure, and the trained probe detector is used to detect an image patch in the 2D X-ray image that contains the ultrasound probe head. In order to train a probe detector, synthetic data can be generated by using a computed tomography (CT) volume of an ultrasound probe. DRR images are generated from the CT volume of the probe in a variety of known poses. Manually annotated training data is also chosen to contain a wide variety of pose orientations and locations in various fluoroscopic images. Additionally, the training data set can include images without a probe to enable to trained probe detector to correctly classify non-object regions. The training method is generic and independent of the probe type. The training data is probe specific and is performed offline prior to online detection.

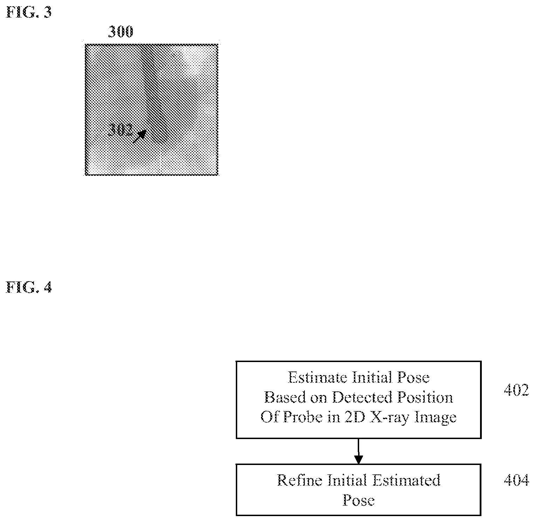

A probabilistic boosting tree (PBT) can be used to train the probe detector from the training data. The PBT can be trained using Haar features extracted image patches in the training data annotated as positive (belonging to the probe) or negative (belonging to tissue other than the probe). At runtime, in order to detect the probe in the received 2D X-ray image, Haar features are extracted from image patches in the 2D X-ray image and the trained PBT classifier determines a probability score for each image patch. The image patch having the highest probability score is determined to be the position of the probe in the fluoroscopic image. FIG. 3 illustrates probe detection in a 2D X-ray image. As shown in FIG. 3, an ultrasound probe 302 is detected in a 2D X-ray image 300 using a trained probe detector.

Returning to FIG. 2, at step 204, a pose of the probe in 3D is estimated. In particular, the pose of the probe relative to the fluoroscopic image is defined by defining the probe's position in 3D (X, Y, Z) and its orientation (roll, pitch, yaw) in the table coordinate system of the C-arm image acquisition device. The detected position of the probe in the 2D fluoroscopic image can be used as a starting point for probe pose estimation using a marginal space learning (MSL) framework.

FIG. 4 illustrates a method for estimating the pose. method of FIG. 4 can be used to implement step 204 of FIG. 2. As illustrated in FIG. 4, at step 402, an initial pose of the probe is estimated based on the detected position of the probe in the fluoroscopic image. In order to estimate the initial pose of the probe, a marginal space learning approach can be used such that the pose is not estimated directly in the full similarity transformation space, but incrementally on projected sample distributions. This enables fast and directed computation. The 2D position of the probe from the probe detection step is used to initialize pose estimation by estimating the X and Y coordinates of the probe. In order to estimate the pose of the probe in 3D, the 3D position (X, Y, Z) of the probe is estimated in a first detection stage, followed by estimation of the position and orientation in a second detection stage.

Learning based techniques are used for each detection stage. This approach treats pose estimation as a classification problem. A training dataset of the probe in different poses is generated offline. The training set can include manually annotated and synthetically generated training data. In a possible implementation, separate PBT classifiers are trained for each detection stage (i.e., position and position-orientation) of the pose estimation. At run time, features (e.g., Haar features) are extracted from the fluoroscopic image and used by the sequence of trained classifiers to estimate the pose of the probe. This approach is fast and provides an initial estimate of the probe's position and orientation.

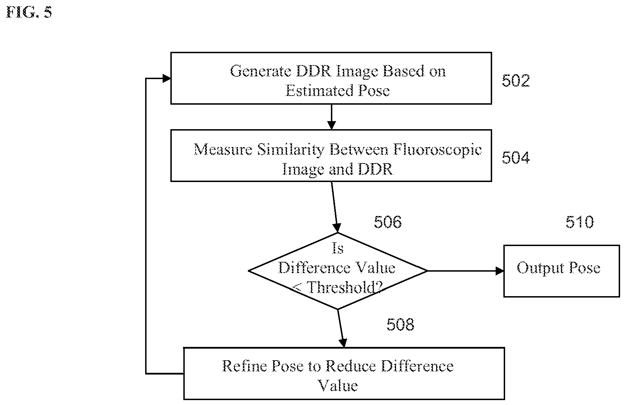

At step 404, the estimated initial pose of the probe is refined. In particular, 2D/3D registration can be used to iteratively refine the pose estimation. FIG. 5 illustrates a method of refining an estimated pose of an ultrasound probe. The method of FIG. 5 can be used to implement step 404 of FIG. 4.

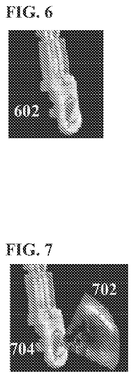

At step 502, a DRR image is generated based on the estimated pose of the probe. A 3D model of the probe is generated offline prior to the cardiac intervention procedure using DynaCT/CT. This model is aligned to the initialized position of the probe in 3D and used to generate a DRR. The DRR produces a representation of the probe which is visually similar to the image captured by the 2D X-ray image. This enables a comparison between the DRR and the 2D X-ray image. At step 504, similarity between the 2D X-ray image and DRR is measured. The similarity may be measured using a difference value that represents an amount of difference between the 2D X-ray image and the DRR. That is, a small difference value indicates that the 2D X-ray image and the DRR are similar. At step 506, it is determined if the difference value between the fluoroscopic image and the DRR is below of given threshold. If the difference value is not below the threshold at step 506, the method proceeds to step 508. At step 508, the pose is refined based on the measured similarity. The pose can be refined by using a local search to determine a new pose that reduces the difference value measured between the 2D X-ray image and the DRR. After the pose is refined, the method returns to step 502 and a new DRR is generated based on the refined pose. The similarity is then measured between the new DRR and the 2D X-ray image at step 504 and the above described steps are repeated until the difference value is below the threshold. If the difference value is below the threshold at step 506, the method proceeds to step 510. At step 510, the pose of the probe is output and the method ends. FIG. 6 illustrates a probe 602 in 3D. The pose of the probe 602 was determined based on the probe detection results in the 2D X-ray image shown in FIG. 3.

Returning to FIG. 2, at step 206, the 3D ultrasound image is registered to the 3D coordinate system of the C-arm image acquisition device based on the estimated pose of the probe in the 3D coordinate system. In particular, the 3D ultrasound image can be mapped to the 3D coordinate system of the C-arm image acquisition device based on the estimated pose of the probe using Ultrasound calibration parameters. The ultrasound device (e.g., the TEE transducer) is calibrated offline prior to the cardiac procedure, resulting in ultrasound calibration parameters. The ultrasound calibration parameters are used to estimate a transform that relates the coordinate system of the 3D ultrasound image to the local coordinate system of the head of the ultrasound probe. Since the pose of the probe is estimated in the 3D coordinate system of the C-arm image acquisition device, the transform maps the 3D ultrasound image to the 3D coordinate system of the C-arm image acquisition device based on the relationship between the head of the ultrasound probe and the 3D ultrasound image. FIG. 7 illustrates a 3D ultrasound image 702 mapped to the 3D local coordinate system of a C-arm image acquisition device based on the pose of the ultrasound probe 704.

In another exemplary embodiment, the registration of the 3D ultrasound image to the coordinate system of the C-arm image acquisition device can be implemented by equipping the ultrasound probe with a position sensor (e.g., an electro-magnetic tracking sensor). The position sensor tracks the position of the ultrasound probe relative to the C-arm image acquisition device, and the tracked position of the ultrasound probe can be used to derive the position of the ultrasound image in the coordinate system of the C-arm image acquisition device.

Returning to FIG. 1, at step 106, a structure of interest is detected in the registered ultrasound image. The structure of interest at least a portion of an anatomical structure that can be used to defined an optimal viewing angle for the X-ray acquisition. Although it is possible for a user to manually mark the structure of interest in the ultrasound image, according to an advantageous embodiment the structure of interest can be detected automatically in the 3D ultrasound image. In a possible implementation, a line of interest, such as a centerline of an anatomical structure, may be detected in the ultrasound image. In another possible implementation, at least three points are detected in the 3D ultrasound image that defined a plane, such as the annulus plane of a heart valve. For example, the centerline of the aortic valve may be detected in the 3D ultrasound image. In another possible implementation, an anatomical structure, such as the aortic valve structure or mitral valve structure, is detected in the 3D ultrasound image.

In an exemplary implementation for TAVI, three "hinge points" of the aortic valve can be automatically detected in the 3D ultrasound image. The three hinge points are the lowest points on the three aortic cusps in the 3D ultrasound image. The three hinge points of the aortic valve define the aortic annulus plane, which is used to determine the C-arm angulation. In addition to the three hinge points, three aortic commissure points and the left and right coronary ostia can also be automatically detected in the 3D ultrasound image to improve the robustness of the detection of the three hinge points. Although it is possible to detect each of the aortic anatomic landmarks separately, the hinge points, commissure points, and coronary ostia can be detected in the 3D image using a hierarchical approach which first detects global object (e.g., bounding box) representing all eight anatomical landmarks (3 hinge points, 3 commissures, and 2 coronary ostia) and then refines each individual anatomic landmark using specific trained landmark detectors. The position, orientation, and scale of the global object is detected by classifiers trained based on annotated training data using marginal space learning (MSL). In order to efficiently localize an object using MSL, parameter estimation is performed in a series of marginal spaces with increasing dimensionality. Accordingly, the idea of MSL is not to learn a classifier directly in the full similarity transformation space, but to incrementally learn classifiers in the series of marginal spaces. As the dimensionality increases, the valid space region becomes more restricted by previous marginal space classifiers. In particular, detection of the global object in the 3D image is split into three stages: position estimation, position-orientation estimation, and position-orientation-scale estimation. A separate classifier is trained based on annotated training data for each of these steps. This object localization results in an estimated transformation (position, orientation, and scale) of the object, and a mean shape of the object is aligned with the 3D volume using the estimated transformation. Boundary delineation of the estimated object shape can then be performed by non-rigid deformation estimation (e.g., using an active shape model (ASM)). The specific landmark detectors for the hinge points, commissure points, and coronary ostia can be trained position detectors that search for the specific landmarks in a region constrained by the detected global object.

In another exemplary implementation for TAVI, a centerline of the aortic root can be detected. For example, the centerline of the aortic root can be detected by detecting 2D circles representing the intersection of the aortic root with horizontal slices or cross sections of the 3D image using a trained circle detector, and tracking the centerpoints of the detected 2D circles. The aortic annulus plane can then be defined as a plane that is perpendicular to the centerline at the aortic annulus point.

In another exemplary implementation for TAVI, the aortic root can be segmented in the 3D ultrasound image using MSL. As described above, in MSL-based segmentation, after estimating the pose (position, orientation, and scale) of an object, the mean shape of the object (e.g., a mean aortic root model generated from training data) is aligned with the estimated pose as an initial estimate of the object shape. After the initial estimate for the pose of the aortic root is detected a learning based boundary model and active shape model can be used to for final boundary delineation of the aortic root. Since an entire aortic root model is segmented, the aortic annulus plane is defined by the segmented aortic root. The segmentation of the aortic root using MSL is described in greater detail in United States Patent Application No. 12/725,679, filed March 17, 2010, entitled "Method and System for Automatic Aorta Segmentation". For interventions involving the mitral valve, the mitral valve can be similarly segmented using MSL to align a mean mitral valve model to the 3D ultrasound image. The segmented mitral valve model defines the annulus plane of the mitral valve in the 3D ultrasound image.

Returning to FIG. 1, at step 108, the C-arm angulation is determined based on the detected structure of interest. The C-arm angulation is dependent on the type of cardiac intervention being performed and is automatically determined based on the structure of interest. For example, for TAVI, the annulus plane of the aortic valve is determined based on the detected structure of interest and a C-arm angulation that is perpendicular to the aortic annulus plane is selected. Similarly, for mitral valve intervention procedures, a C-arm angulation that is perpendicular to the annulus plane of the mitral valve can be selected.

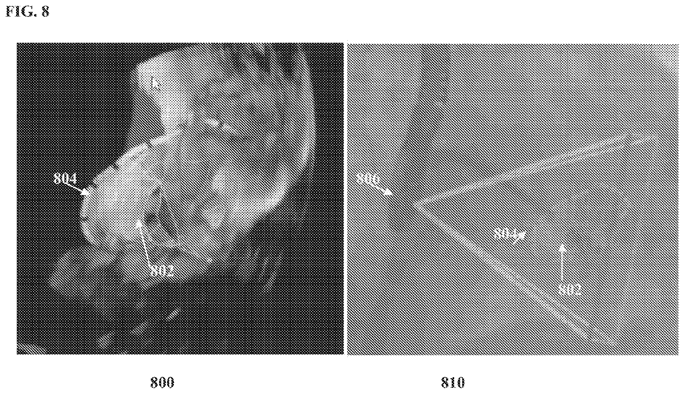

FIG. 8 illustrates exemplary C-arm angulation planning based on the annulus plane of the mitral valve. As shown in FIG. 8, image 800 shows a detected mitral valve model 802 including the mitral valve annulus plane 804 in a 3D ultrasound image. Image 810 shows an X-ray image of the ultrasound probe 806 and the registered mitral valve model 802 including the mitral valve annulus plane 804. An optimal C-arm angulation for a mitral valve intervention can be achieved by rotating the C-arm image acquisition device to be perpendicular to the mitral annulus plane 804.

Returning to FIG. 1, at step 110, the C-arm image acquisition device is oriented to the determined angulation. In a possible implementation, the C-arm can be oriented to the determined angulation automatically. For example, the determined angulation can be provided to a controller of the C-arm image acquisition device, which can then automatically rotate the C-arm to the determined angulation. In another possible implementation, the C-arm can be oriented semi-automatically. For example, the determined angulation can be output to the user (e.g., by displaying the determined angulation on a display of the device), and the user can then rotate the C-arm to the determined angulation. The angulation can then be kept by the user or modified interactively by the user.

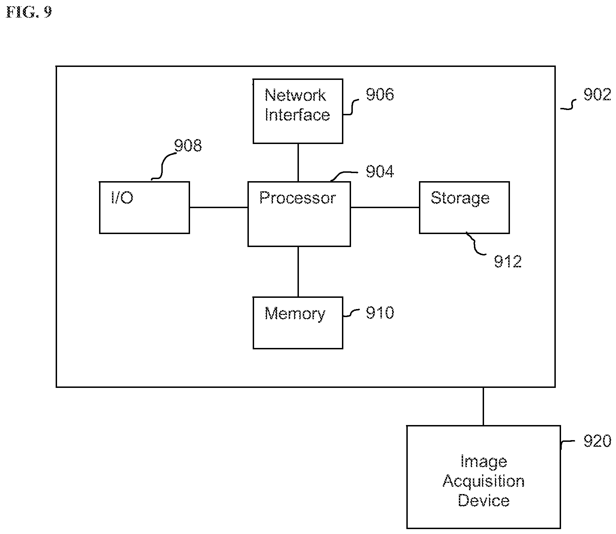

The above-described methods for determining an angulation of a C-arm image acquisition system may be implemented on one or more computers using well-known computer processors, memory units, storage devices, computer software, and other components. A high level block diagram of such a computer is illustrated in FIG. 9. Computer 902 contains a processor 904 which controls the overall operation of the computer 902 by executing computer program instructions which define such operation. The computer program instructions may be stored in a storage device 912, or other computer readable medium (e.g., magnetic disk, CD ROM, etc.) and loaded into memory 910 when execution of the computer program instructions is desired. Thus, the steps of the methods of FIGS. 1, 2, 4, and 5 may be defined by the computer program instructions stored in the memory 910 and/or storage 912 and controlled by the processor 904 executing the computer program instructions. An image acquisition device 920 can be connected to the computer 902 to input images to the computer 902. For example the image acquisition device 920 may be a C-arm image acquisition system capable of acquiring X-ray images and inputting the X-ray images to the computer 902. The image acquisition device 920 can also include an ultrasound probe, such as a TEE or ICE ultrasound probe, capable of acquiring 3D ultrasound images and inputting the ultrasound images to the computer 902. It is possible to implement the image acquisition device 920 and the computer 902 as one device. It is also possible that the image acquisition device 920 and the computer 902 communicate wirelessly through a network. The computer 902 also includes one or more network interfaces 906 for communicating with other devices via a network. The computer 902 also includes other input/output devices 908 that enable user interaction with the computer 902 (e.g., display, keyboard, mouse, speakers, buttons, etc.). One skilled in the art will recognize that an implementation of an actual computer could contain other components as well, and that FIG. 9 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws.

## Claims

1. An apparatus (902) for determining an angulation of a C-arm image acquisition system for a cardiac intervention, comprising:
means for receiving a 3D ultrasound image including a cardiac region using an ultrasound probe at the beginning of the cardiac procedure or directly from the ultrasound probe in real time during the cardiac procedure, wherein the 3D ultrasound image comprises a transesophageal echo, TEE, or intracardiac echo, ICE;
means for registering the 3D ultrasound image to a 3D coordinate system of the C-arm image acquisition system;
means for detecting a cardiac structure of interest in the registered 3D ultrasound image; and
means for automatically determining an angulation of the C-arm image acquisition system based on the detected structure of interest in the registered 3D ultrasound image;
wherein the means for registering the 3D ultrasound image to a 3D coordinate system of the C-arm image acquisition system comprises:
means for detecting a position of an ultrasound probe in a 2D x-ray image acquired by the C-arm image acquisition device (920);
means for estimating a pose of the ultrasound probe in the 3D coordinate system of the C-arm image acquisition device (920) based on the detected position of the ultrasound probe in the 2D x-ray image using a probabilistic boosting tree; and
means for registering the 3D ultrasound image to the 3D coordinate system of the C-arm image acquisition device (920) based on the estimated pose of the ultrasound probe in the 3D coordinate system of the C-arm image acquisition device (920).

2. The apparatus of claim 1, wherein the means for registering the 3D ultrasound image to a 3D coordinate system of the C-arm image acquisition system comprises:
means for tracking a position of an ultrasound probe in the 3D coordinate system of the C-arm image acquisition device (920); and
means for registering the ultrasound image to the 3D coordinate system of the C-arm image acquisition device (920) based on the tracked position of the ultrasound probe.

3. The apparatus of claim 1, wherein the cardiac intervention is a transcatheter aortic valve implantation (TAVI) procedure and the means for detecting a cardiac structure of interest in the registered 3D ultrasound image comprises: means for detecting an aortic annulus plane in the registered 3D ultrasound image,
in particular wherein the means for determining an angulation of the C-arm image acquisition system based on the detected structure of interest in the registered 3D ultrasound image comprises: means for determining an angulation of the C-arm image acquisition device (920) that is perpendicular to the detected aortic annulus plane.

4. The apparatus of claim 1, wherein the cardiac intervention is a mitral valve intervention and the means for detecting a cardiac structure of interest in the registered 3D ultrasound image comprises: means for detecting an annulus plane of the mitral valve in the registered 3D ultrasound image,
in particular wherein the means for determining an angulation of the C-arm image acquisition system based on the detected structure of interest in the registered 3D ultrasound image comprises: means for determining an angulation of the C-arm image acquisition device (920) that is perpendicular to the detected annulus plane of the mitral valve.

## Patentansprüche

1. Vorrichtung (902) zum Bestimmen einer Abwinkelung eines Bilderfassungssystems mit C-Bogen für eine kardiale Intervention, umfassend:
Mittel zum Empfangen eines 3D-Ultraschallbildes, das eine kardiale Region einschließt, unter Verwendung einer Ultraschallsonde zu Beginn des kardialen Eingriffs oder direkt von der Ultraschallsonde in Echtzeit während des kardialen Eingriffs, wobei das 3D-Ultraschallbild ein transösophageales Echo, TEE, oder intrakardiales Echo, ICE, umfasst;
Mittel zum Registrieren des 3D-Ultraschallbildes an einem 3D-Koordinatensystem des Bilderfassungssystems mit C-Bogen;
Mittel zum Detektieren einer interessierenden kardialen Struktur in dem registrierten 3D-Ultraschallbild; und
Mittel zum automatischen Bestimmen einer Abwinkelung des Bilderfassungssystems mit C-Bogen basierend auf der interessierenden detektierten Struktur in dem registrierten 3D-Ultraschallbild;
wobei das Mittel zum Registrieren des 3D-Ultraschallbildes an einem 3D-Koordinatensystem des Bilderfassungssystems mit C-Bogen umfasst:
Mittel zum Detektieren einer Position einer Ultraschallsonde in einem 2D-Röntgenbild, das durch die Bilderfassungsvorrichtung mit C-Bogen (920) erfasst wurde;
Mittel zum Schätzen einer Stellung der Ultraschallsonde in dem 3D-Koordinatensystem der Bilderfassungsvorrichtung mit C-Bogen (920) basierend auf der detektierten Position der Ultraschallsonde in dem 2D-Röntgenbild unter Verwendung eines probabilistischen Boosting-Baums; und
Mittel zum Registrieren des 3D-Ultraschallbildes an dem 3D-Koordinatensystem der Bilderfassungsvorrichtung mit C-Bogen (920) basierend auf der geschätzten Stellung der Ultraschallsonde in dem 3D-Koordinatensystem der Bilderfassungsvorrichtung mit C-Bogen (920).

2. Vorrichtung nach Anspruch 1, wobei das Mittel zum Registrieren des 3D-Ultraschallbildes an einem 3D-Koordinatensystem des Bilderfassungssystems mit C-Bogen umfasst:
Mittel zum Verfolgen einer Position einer Ultraschallsonde in dem 3D-Koordinatensystem der Bilderfassungsvorrichtung mit C-Bogen (920); und
Mittel zum Registrieren des Ultraschallbildes an dem 3D-Koordinatensystem der Bilderfassungsvorrichtung mit C-Bogen (920) basierend auf der verfolgten Position der Ultraschallsonde.

3. Vorrichtung nach Anspruch 1, wobei die kardiale Intervention ein Eingriff zur Transkatheter-Aortenklappen-Implantation (TAVI) ist, und das Mittel zum Detektieren einer interessierenden kardialen Struktur in dem registrierten 3D-Ultraschallbild umfasst:
Mittel zum Detektieren einer Aortenannulusebene in dem registrierten 3D-Ultraschallbild,
wobei insbesondere das Mittel zur Bestimmung einer Abwinkelung des Bilderfassungssystems mit C-Bogen basierend auf der detektierten interessierenden Struktur in dem registrierten 3D-Ultraschallbild umfasst:
Mittel zum Bestimmen einer Abwinkelung der Bilderfassungsvorrichtung mit C-Bogen (920), die senkrecht zu der detektierten Aortenannulusebene ist.

4. Vorrichtung nach Anspruch 1, wobei die kardiale Intervention eine Mitralklappenintervention ist, und das Mittel zum Detektieren einer interessierenden kardialen Struktur in dem registrierten 3D-Ultraschallbild umfasst:
Mittel zum Detektieren einer Annulusebene der Mitralklappe in dem registrierten 3D-Ultraschallbild,
wobei insbesondere das Mittel zum Bestimmen einer Abwinkelung des Bilderfassungssystems mit C-Bogen basierend auf der interessierenden detektierten Struktur in dem registrierten 3D-Ultraschallbild umfasst:
Mittel zum Bestimmen einer Abwinkelung der Bilderfassungsvorrichtung mit C-Bogen (920), die senkrecht zu der detektierten Annulusebene der Mitralklappe ist.

## Revendications

1. Appareil (902) destiné à déterminer une angulation d'un système d'acquisition d'images sur arceau pour une intervention cardiaque, comprenant :
des moyens pour recevoir une image ultrasonore 3D comportant une région cardiaque en utilisant une sonde à ultrasons au début de la procédure cardiaque ou directement depuis la sonde à ultrasons en temps réel pendant la procédure cardiaque, l'image ultrasonore 3D comprenant un écho transœsophagien, TEE, ou un écho intracardiaque, ICE ;
des moyens pour aligner l'image ultrasonore 3D sur un système de coordonnées 3D du système d'acquisition d'images sur arceau ;
des moyens pour détecter une structure cardiaque d'intérêt dans l'image ultrasonore 3D alignée ; et
des moyens pour déterminer automatiquement une angulation du système d'acquisition d'images sur arceau sur la base de la structure d'intérêt détectée dans l'image ultrasonore 3D alignée ;
dans lequel les moyens pour aligner l'image ultrasonore 3D sur un système de coordonnées 3D du système d'acquisition d'images sur arceau comprennent :
des moyens pour détecter une position d'une sonde à ultrasons dans une image aux rayons X 2D acquise par le dispositif d'acquisition d'images sur arceau (920) ;
des moyens pour estimer une pose de la sonde à ultrasons dans le système de coordonnées 3D du dispositif d'acquisition d'images sur arceau (920) sur la base de la position détectée de la sonde à ultrasons dans l'image aux rayons X 2D en utilisant un arbre de renforcement probabiliste ; et
des moyens pour aligner l'image ultrasonore 3D sur le système de coordonnées 3D du dispositif d'acquisition d'images sur arceau (920) sur la base de la pose estimée de la sonde à ultrasons dans le système de coordonnées 3D du dispositif d'acquisition d'images sur arceau (920).

2. Appareil de la revendication 1, dans lequel les moyens pour aligner l'image ultrasonore 3D sur un système de coordonnées 3D du système d'acquisition d'images sur arceau comprennent :
des moyens pour suivre une position d'une sonde à ultrasons dans le système de coordonnées 3D du dispositif d'acquisition d'images sur arceau (920) ; et
des moyens pour aligner l'image ultrasonore sur le système de coordonnées 3D du dispositif d'acquisition d'images sur arceau (920) sur la base de la position suivie de la sonde à ultrasons.

3. Appareil de la revendication 1, dans lequel l'intervention cardiaque est une procédure d'implantation transcathéter de valvule aortique (TAVI) et les moyens pour détecter une structure cardiaque d'intérêt dans l'image ultrasonore 3D alignée comprennent : des moyens pour détecter un plan d'anneau aortique dans l'image ultrasonore 3D alignée, en particulier dans lequel les moyens pour déterminer une angulation du système d'acquisition d'images sur arceau sur la base de la structure d'intérêt détectée dans l'image ultrasonore 3D alignée comprennent : des moyens pour déterminer une angulation du dispositif d'acquisition d'images sur arceau (920) qui est perpendiculaire au plan d'anneau aortique détecté.

4. Appareil de la revendication 1, dans lequel l'intervention cardiaque est une intervention sur la valvule mitrale et les moyens pour détecter une structure cardiaque d'intérêt dans l'image ultrasonore 3D alignée comprennent : des moyens pour détecter un plan d'anneau de la valvule mitrale dans l'image ultrasonore 3D alignée,
en particulier dans lequel les moyens pour déterminer une angulation du système d'acquisition d'images sur arceau sur la base de la structure d'intérêt détectée dans l'image ultrasonore 3D alignée comprennent : des moyens pour déterminer une angulation du dispositif d'acquisition d'images sur arceau (920) qui est perpendiculaire au plan d'anneau détecté de la valvule mitrale.
